# EUROPEAN PATENT APPLICATION

(11) **EP 2 070 554 A1**
(43) Date of publication of application: **17.06.2009**
(21) Application number: 08425012.5
(22) Date of filing: 09.01.2008
(51) Int. Cl.: A61L 9/12

(54) **Device to seal lock the containers of fragrances for the environment**

(30) Priority: 10.12.2007 IT PI20070136
(71) Applicant: Deo Promotion S.r.l., 42015 Correggio (RE) (IT)
(72) Inventor: Artoni, Simone, 42015 Correggio (RE) (IT)
(74) Representative: Turini, Laura

(57) **Abstract**

A sealing device (10) for fragrances for the environment that has a number of holes (13). Diffuser elements (4a) can be inserted into the specific holes (13) for a certain portion of their entire length. The joint made between the diffuser elements and the holes, happens with interference, and therefore, the same holes become sealed. In this manner, once the sealing device (10) is inserted with the diffuser elements (4) on the mouth (2a) of the bottle §(2), so that the diffuser elements are partially dipped into the liquid (3), a completely sealed fragrance device is obtained. Any kind of crash that may cause the overturn of the fragrance device, will not be the cause of any leaking of the liquid.

## Description

### Technical field

The undertaken invention regards the technical field relative to the production of deodorants and fragrances in general, in particular it refers to the production of fragrances specific for the environment.

### Background art

As it is well known in the field relative to the production of fragrances and deodorants for the environment, there are many types of devices for releasing the fragrance essence in the surrounding environment all different from each other, both in terms of fragrance that in the device used. In some cases, for example, these devices are typically more technological because they have time systems that allow the automatic release in predetermined time-slot of the fragrance in the surrounding environment.

In other cases, less complex system are found, but which compensate better to the embellishment and interior decoration of the environment, as the rooms of a house, shop or similar and moreover they allow in a simple and fast way to change the fragrance according to someone's taste and circumstances. In this case, between the different types of common fragrances devices, there is a particular category that uses specifically fragrance liquids of diverse kind, as for example oils or essence. The diffusion to the surrounding environment of these fragrances can be made in diverse manners.
or example, a typical way to spread into the surrounding environment the perfume of the liquid fragrance, is the one that uses the absorption of the same liquid through specific means of diffusion as sticks or similar. In particular, a portion of the sticks are inserted inside the container of the liquid and are in direct contact with the same liquid. In this way, the sticks, after a while, absorb all the fragrance liquid, letting it rise slowly along their whole length, allowing this way the spreading in the surrounding environment with a slow discharge.

In other cases it is possible to help the spreading of the fragrance integrating, to the sticks inserted in the container of the fragrance liquid, means of heating which help the evaporation of the liquid in the air. in this case the container could be placed above a support which contains a small candle to warm up.
however the container with the spreading sticks is not exempted from some inconvenient. For example, both in the case the container is hit for accident and in the case the supporting base is not stable, there is a possibility that the same container turns over and the whole liquid inside of it pours. The uneasiness in this case are a lot, as the fragrance oils or liquids are in general corrosive for the furniture and there is a risk that they get permanently stained.

Moreover in the case the liquid is spread into the environment by using simultaneously the heat and the absorption through sticks, there is a risk of searing parts of the body.

### Disclosure of invention

It is the aim of this invention to give a closing device for the above mentioned fragrances that prevents the accidental leak of the liquid during the usage.
it is also the aim of this invention to give a closing system for the fragrances that does not interfere in any way with the usual spreading of the fragrance into the external environment.

These and other aims are obtained through this device able to collect and maintain in a stable position inside the fragrance device the means of spreading of the fragrance, as deodorant sticks or sticks, into the environment.

In this way, a portion of the length of the sticks can be inserted inside the container, which is the main body of the fragrance device, and remain clustered and stable at their position. The seal device comprises:
- A longitudinal section containing at least a via hole. This via hole extends to the whole length of the mentioned longitudinal section and is able to collect and contain these means of fragrance spreading inserted for the mentioned portion of their whole length inside the mentioned container in a way that these means of spreading do not tip over the mouth of the mentioned container.
- A lesser surface that has a predetermined cross-section length that allows to have a strict connection with the mouth of the mentioned container.
- An upper surface that has a predetermined length of the cross- section and through which it is possible to realize the insertion of the mentioned fragrance diffusers through the above mentioned via hole of the mentioned longitudinal section, in which the via hole of the sealing device has a diameter equivalent or close to the ones of the means of diffusion in a way that the joining between these means of diffusion and the via hole happens with interference.

In this manner, in the case accidentally the fragrance device is turned over, the spilling of the whole liquid contained inside the same fragrance device is avoided.

Moreover, when the upward pushing is strong, there is a risk that the diffuser elements of the container fall out. For this reason it can be requested that the value of the interference from joining is as much as to impede at least the motion of the diffuser elements partially dipped into the liquid for a portion of their entire length in respect to the sealing device.

### Brief description of Drawings

In Fig. (1) it is represented in a sketch the Prior Art relative to a kind of fragrance device for the environment. Some fragrance liquid (3), as for example oil, can be contained inside an appropriate container (2). With the aim to obtain a gradual release of the fragrance of the oil (3) into the surrounding environment, are commonly used some diffuser elements (4). These are inserted inside the container (2) through its mouth (2a) in a way that a portion of their entire length is dipped into the liquid.

The diffuser elements that can be used could be common sticks (4) made in wood or, alternatively, different types of material. Indeed the sole important characteristic that the material used shall have, is the ability to absorb the liquid in which it is dipped into. in this manner, slowly the sticks (4) absorb the liquid in which they are dipped into and release the fragrance into the surrounding environment through their portion that is not dipped and that is out from the mouth (2a).

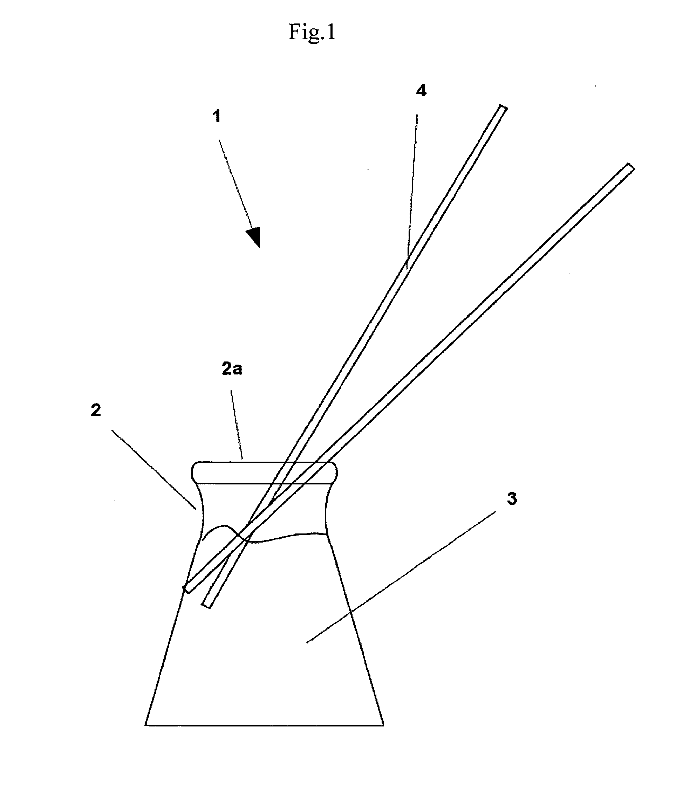

The first inconvenient that is immediately noticed when using this simple fragrance device for the environment is that, until the sticks have not absorbed a certain amount of liquid, it is affected by a really elevated floating force of the liquid which is sufficient to make the liquid spill out from the container (2), and turn over on the leaning plane of the same bottle (Fig.1).

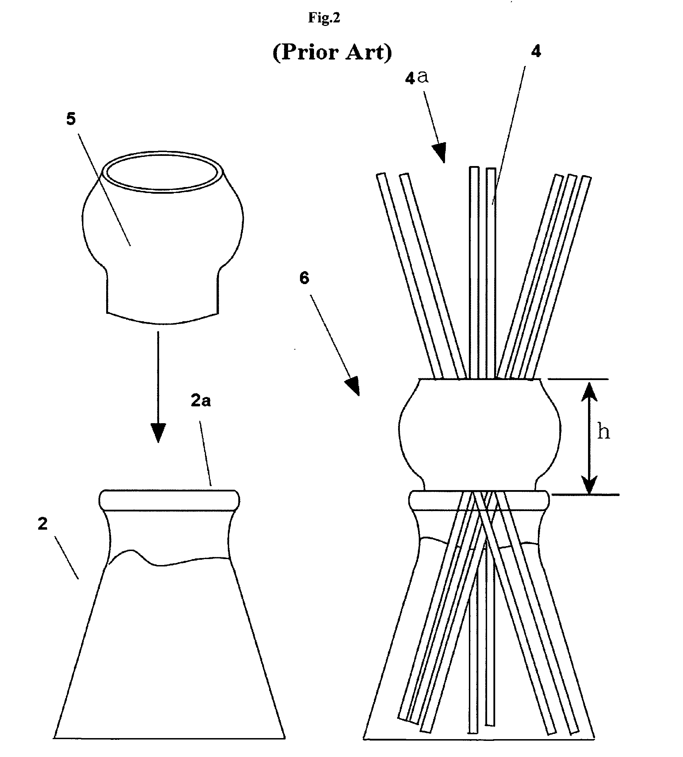

With the aim to get over this inconvenient, it is usually used a sort of sealing device (5), which is inserted on the mouth (2a) exactly as a cap (Fig.2).The sealing device (5) makes this way a certain increase of sealing height h which helps the containment of the group of sticks (4a) in position avoiding the spilling from the bottle, at least until all the sticks are totally soaked.

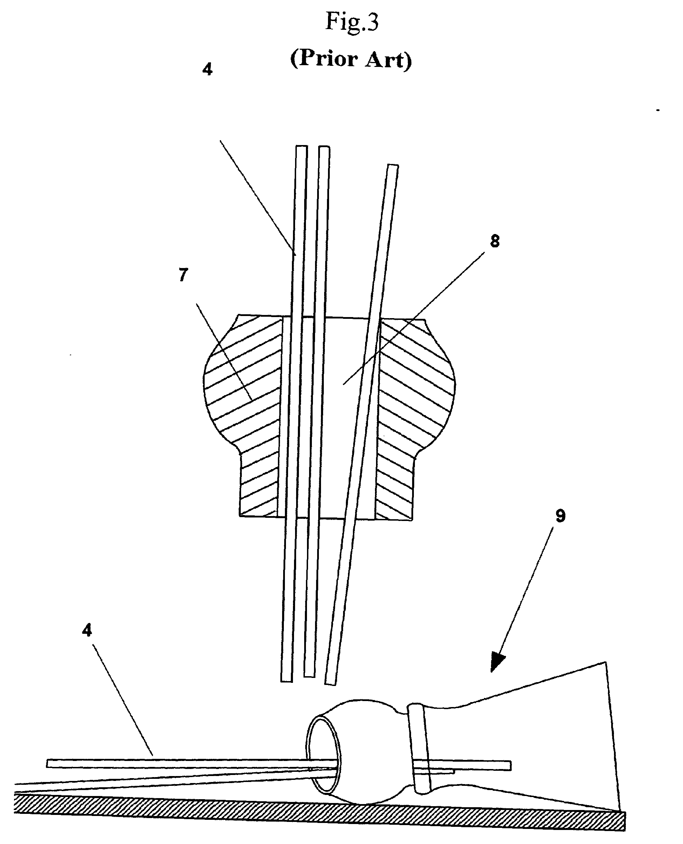

As it is clearly shown in fig. 3, the via hole (8) of the sealing device (5) has a certain dimension to allow the containment of a certain number of sticks (4). As a consequence, an accidental hit of the fragrance device (9) which cause an overturn, provokes the complete spilling of the liquid contained in it, permanently damaging the furnisher or support on which it leans on.

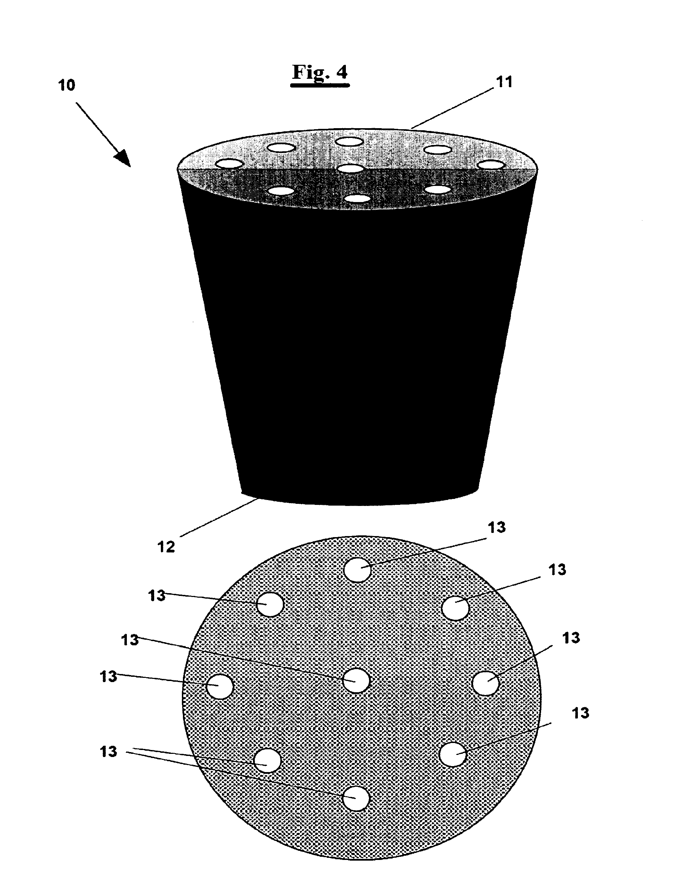

n fig. 4 is described an alternative solution according to the invention and able to solve all the above mentioned inconvenient.
in particular, the new sealing device (10) is made by an upper surface (11) and a lower surface (12)and from a number of via holes (13) that go through all its section.

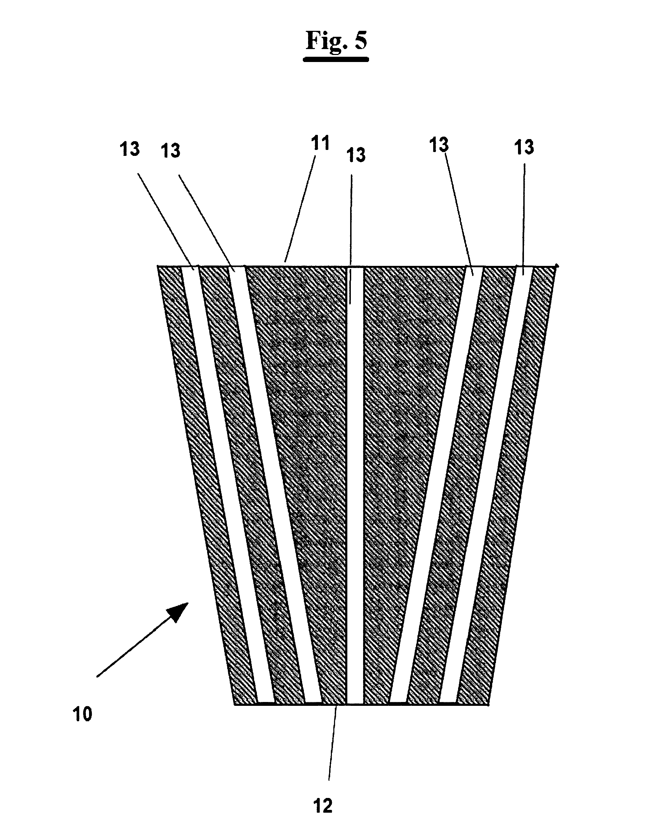

As described in detail in fig. 5, the holes (13) cross the entire section of the sealing device (10) in a way to connect the upper surface (11) with the lower one (12).

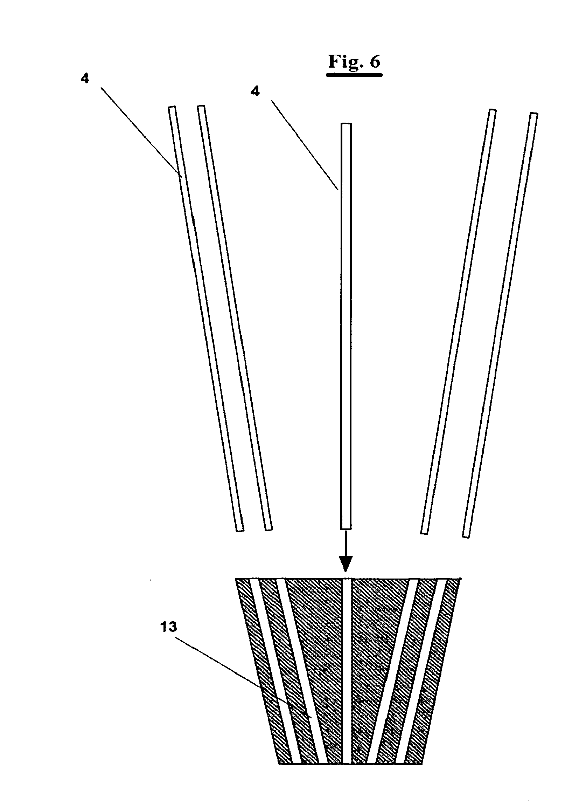

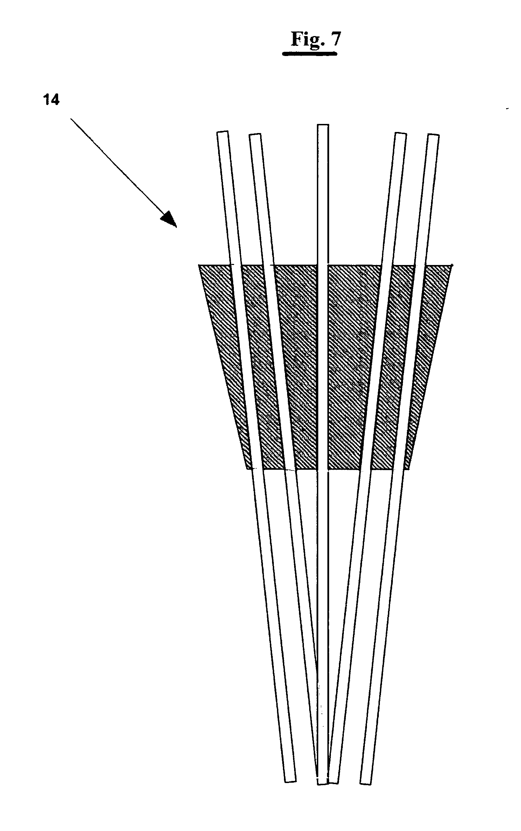

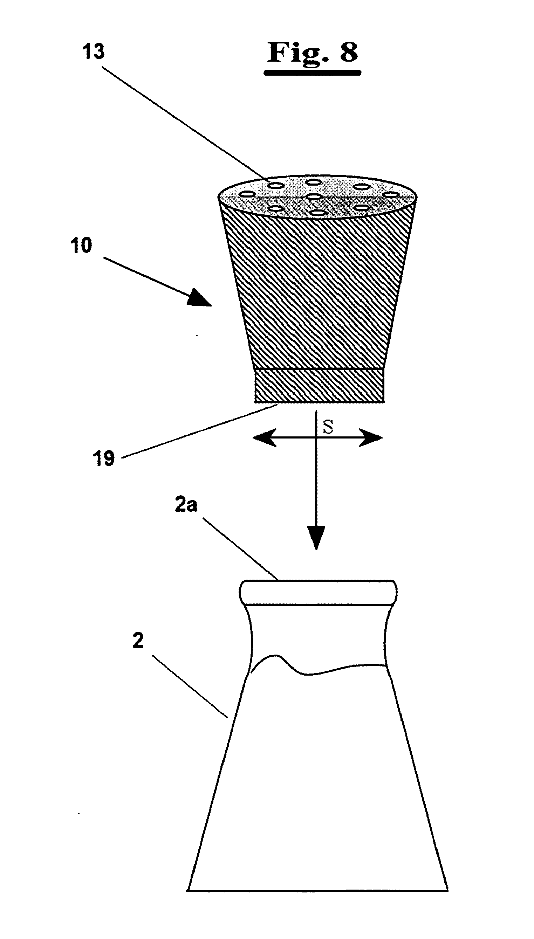

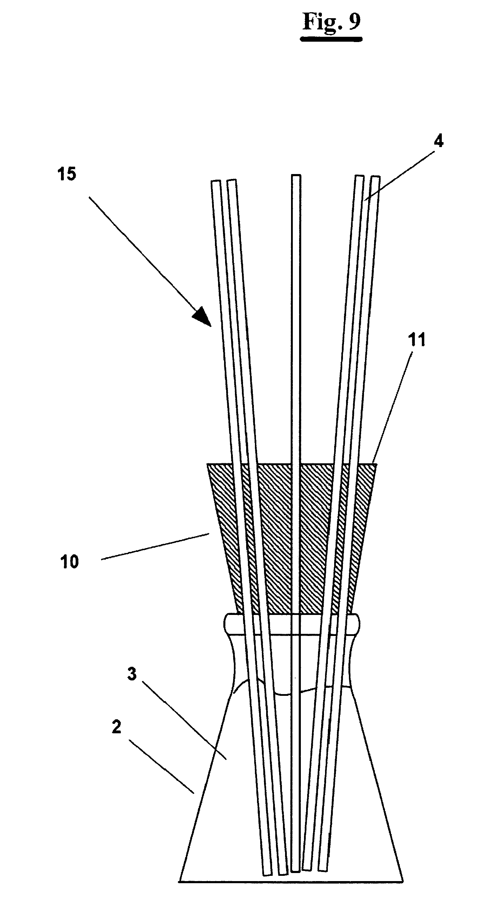

The feature of this sealing device is that it utilizes each hole (13) as a site for one respective diffuser element (4) (Fig. 6 and 7). In particular, making the present holes (13) on the sealing device (10) with a diameter equivalent or almost to the one of the used diffuser elements (4), it is possible to make a strict connection with interference between the diffuser elements (4) and the holes (13) of the same sealing device, obtaining this way a sole and rigid body (14).
in this manner, once the sealing device (10) is put on the bottle (2), as indicated in fig. 8, and the diffuser elements are inserted inside the specific via holes (13) through the upper surface (11) of the same device (10), it is practically obtained a fragrance device (15) completely sealed (Fig.9). Of course it is possible to assemble the diffuser elements (4) to the sealing device (10) before to get a sole body (14), and only after assemble the whole to the bottle (2).

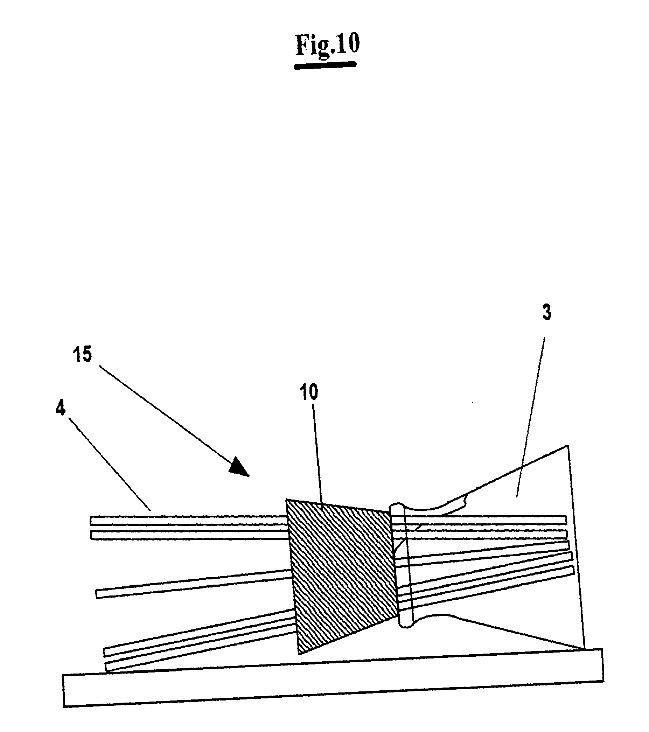

According to the present invention all the advantages are shown clearly. First of all, as indicated in fig.9, the interference found when connecting the diffuser elements (4)to the holes of the sealing device (10), blocks and bind the same diffuser elements (4), which become completely unable to go up and flow over the bottle (2), despite the upward shove caused by their floating inside the liquid (2a). Moreover (Fig. 10) in the case there is an accidental overturn of the bottle caused by an impact or something else, the leaking of the liquid contained inside is totally impeded, as it is impeded the overturning of the diffuser elements, being them blocked inside the holes (13). This simple device allows to safeguard and protection of all the possible supporting surface of the fragrance device (15) during its using.

According to the preferred pattern realization of the invention, it is possible to make the sealing device (10)with the lower surface (16)that has threads (17) (Fig.11). in this way, it is possible to screw the sealing device (10)directly on the mouth of the fragrance bottle (2), guaranteeing a safer and effective seal(Fig. 12 and 13).

Without moving apart from the inventive step, it could be also possible to use sealing device (10) where the connecting system with the mouth of the bottle (2)is made in a different way. In fig. 8 and 9 it is described a sealing device (10) where the lower surface (19) has the cross- section length s in such a manner to be inserted inside the mouth (2a)of the bottle (2).

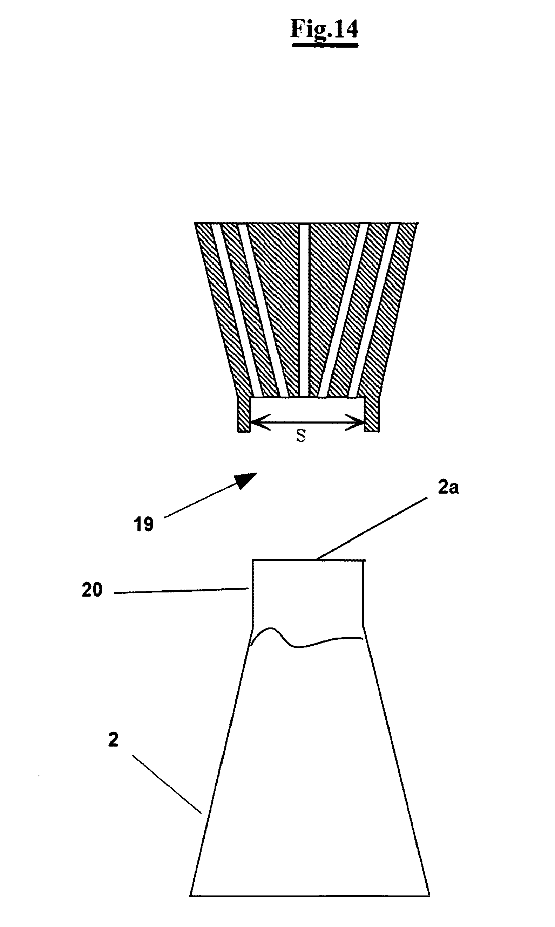

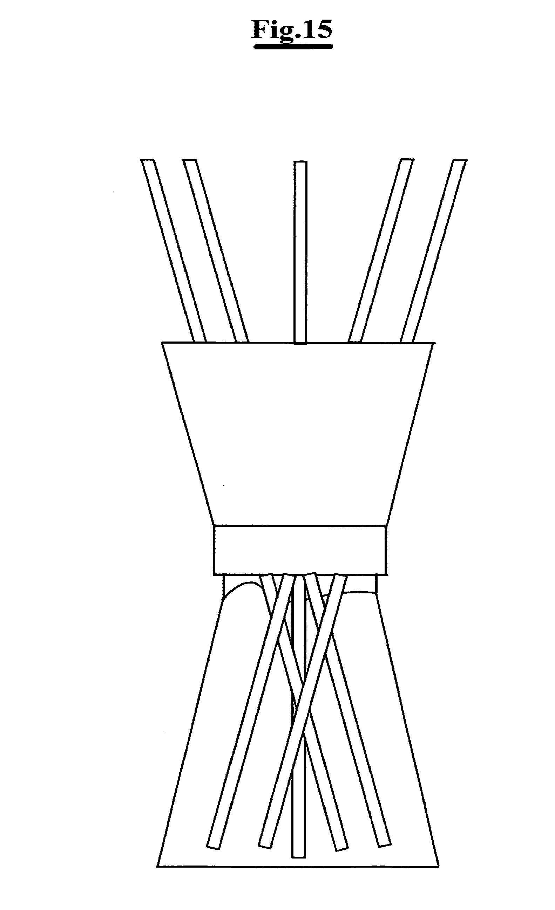

In other cases, it is possible to make a sealing device (10) where the lower surface (19) has the cross- section length in such a manner to be inserted with interference around the mouth (20)of the bottle (2) (Fig. 14 and 15).

Without moving apart from the inventive step, it could be also possible to make a sealing device (10) in a way that the lower surface (19) has the cross- section length s in such a manner to be inserted inside the mouth (2a)of the bottle (2), being this surface (19) supplied with an external thread that allows the screw the mentioned sealing device (10) directly on an internal thread found inside the mouth (2a) of the fragrance bottle (2).

The above description of a specific pattern realization is able to show the invention from the conceptive point of view, in a way that others, by using the prior art, can modify and/or adapt in different applications this specific pattern realization without any further research and without going apart from the inventive concept, and, therefore, it is intended that these adaptations and transformations will be considered as equivalent to this specific realization. The means and materials to make the many described functions can be of various nature without exiting the area of the invention. it is intended that the expressions or the terminology used have a simple descriptive aim and therefore not limiting.

### Brief description of drawings

Further features and advantages of the sealing device, according to the invention, will be clearer with the description of one of its pattern realization that follows, made to illustrate but not limit, with reference to the annexed drawings, in which:
- Fig. 1 and 2 represent a prospective view of a fragrance for the environment according to the prior art.
- Fig. 3 represent a section and a prospective view of a specific fragrance device for the environment according to the prior art.
- Fig. 4 represents a prospective view of the sealing device according to the invention.
- Fig. 5, 6 and 7 represent a section of the sealing device according to the invention.
- Fig. 8, 9 and 10 represent a prospective view according to the invention.

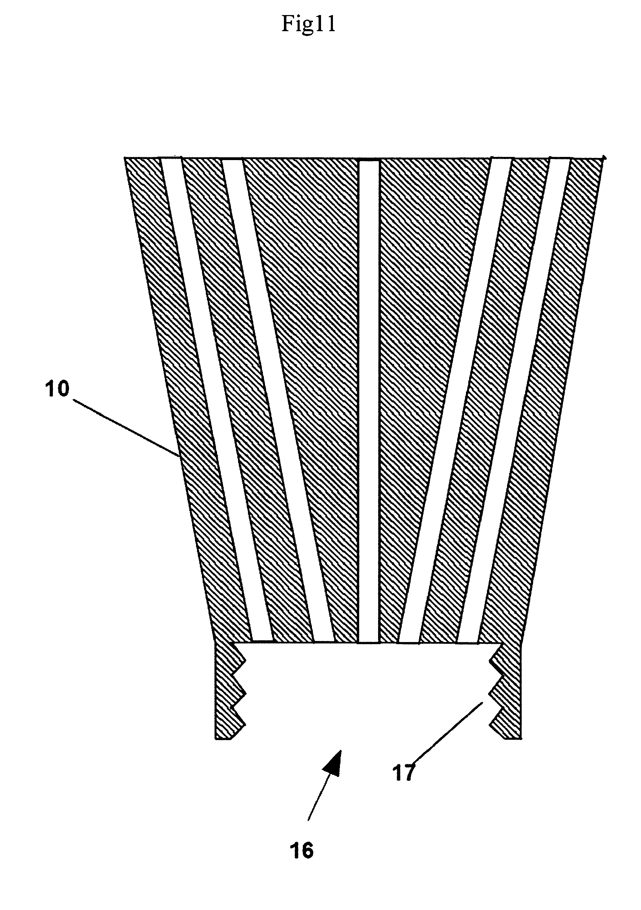

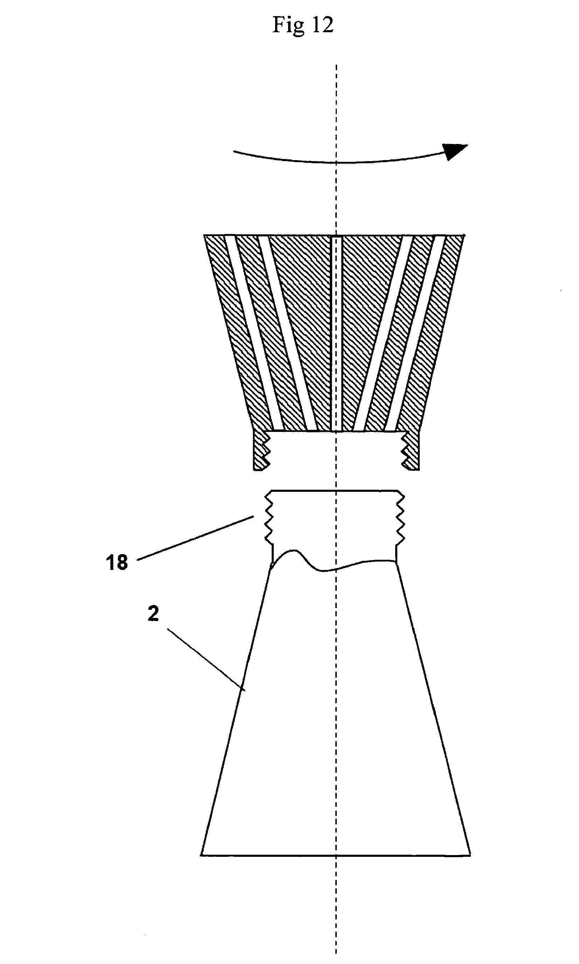

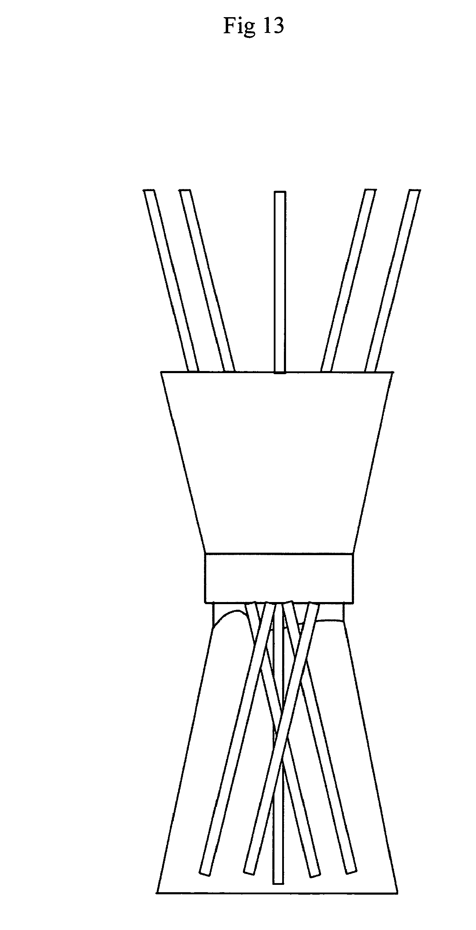

- Fig. 11, 12 and 13 represent a prospective view according to the preferred configuration of the invention.

Fig. 14, 15 and represent a prospective view according to alternative solutions.

## Claims

1. A sealing device made to collect and maintain in a stable position means of diffusion of fragrances for the environment as deodorant sticks, sticks or similar, useful to be inserted for a portion of their entire length inside the containers of the fragrances, as bottle, these containers are supplied with a mouth and contain deodorant liquids and the mentioned containment device comprising:
- A longitudinal section containing at least a via hole, which extends to the whole length of the mentioned longitudinal section and is able to collect and contain these means of fragrance diffusers inserted for the mentioned portion of their whole length inside the mentioned container in a way that these means of spreading do not tip over the mouth of the mentioned container.
- A lesser surface that has a predetermined transversal section length that allows to have a strict connection with the mouth of the mentioned container.
a greater surface that has a predetermined cross-section length and through which it is possible to make the insertion of these diffusers of fragrance through the mentioned via hole of the mentioned longitudinal section, **characterized by the fact that** at least one of the holes of the mentioned sealing device has a diameter equivalent or close to the diameter of the mentioned means of spreading in such a manner that the joint between these means of spreading and the via hole happens with interference.

2. Containment device made to collect and maintain the stable position of the means of spreading of the fragrances for the environment, according to claim 1, in which the value of the above mentioned interference of the mentioned joint is such as to impede at least the motion of these diffuser elements from the mentioned sealing element caused by the shove of the liquid in which these diffuser elements are partially dipped for a portion of their entire length.

3. Device according to one or more of the previous claims in which the sealing device (10) comprises a lower surface (16) that has an internal thread (17) in a way to screw this sealing device (10) directly to an external thread obtained on the mouth of the fragrance bottle (2).

4. Device according to one or more of the previous claims in which the sealing device (10) comprises a lower surface (19) that has a cross-section length s long as much as it can be inserted inside the mouth (2a) of the bottle (2), being this surface (19) supplied with an external thread that allows to screw this sealing device (10) directly to an internal thread obtained inside the mouth (2a) of the fragrance bottle (19).

5. Device according to one or more of the previous claims in which the sealing device (10) comprises a lower surface (19) that has a cross-section length s long as much as it is inserted inside the mouth (2a) of the bottle (2) with interference.

6. Device according to one or more of the previous claims in which the sealing device (10) comprises a lower surface (19) that has a cross-section length s long as much as it is inserted around the mouth (20) of the bottle (2) with interference.

## Amended claims

### Amended claims in accordance with Rule 137(2) EPC.

**1.** A fragrance device (1) for the environment comprising:
- A container (2) for a fragrance liquid (3);
- At least a diffuser element (4), such as deodorant stick, stick or similar, and adapted to be inserted into the container such that a portion of its entire length is dipped into the liquid;
- A sealing device (10) comprising an upper (11) and a lower surface (12; 16; 19) and connected to the mouth (2a) of said container (2) via said lower surface (12; 16; 19), said lower surface (12; 16; 19) having a predetermined transversal section such that to allow a removable rigid connection with said mouth (2a) of said container;
and wherein said scaling device (10) further comprises:
- At least a hole (13), said hole (13) crossing the entire longitudinal section of said sealing device (10) in a way to connect said upper surface (11) with said lower surface (12; 16; 19);
- Said upper surface (11) having a predetermined transversal cross section length and through which it is possible to make the insertion of said at least a diffuser element (4) through said hole (13) of the said longitudinal section such that said diffuser element (4) do not overturn from the mouth of said container (2),
and **characterized by** the fact that said at least a hole (13) of said sealing device (10) has a diameter equivalent or close to the diameter of said diffuser element (4) in such a manner that the coupling between said diffuser element and said hole is with interference.

**2.** Device, according to claim 1, in which the value of the above mentioned interference of the mentioned coupling is such as to impede at least the motion of these diffuser elements (4) from the mentioned sealing device (10) caused by the shove of the liquid in which these diffuser elements arc partially dipped for a portion of their entire length.

**3.** Device according to one or more of the previous claims in which the sealing device (10) comprises a lower surface (16) that has an internal thread (17) in a way to screw this scaling device (10) directly to an external thread obtained on the mouth of the container (2).

**4.** Device according to one or more of the previous claims in which the sealing device (10) comprises a lower surface (19) that has a cross-section length s long as much as it can be inserted inside the mouth (2a) of the bottle (2), being this surface (19) supplied with an external thread that allows to screw this sealing device (10) directly to an internal thread obtained inside the mouth (2a) of the fragrance bottle (19).

**5.** Device according to one or more of the previous claims in which the sealing device (10) comprises a lower surface (19) that has a cross-section length s long as much as it is inserted inside the mouth (2a) of the bottle (2) with interference.

**6.** Device according to one or more of the previous claims in which the sealing device (10) comprises a lower surface (19) that has a cross-section length s long as much as it is inserted around the mouth (20) of the bottle (2) with interference.
